# EUROPEAN PATENT APPLICATION

(11) **EP 2 258 863 A1**
(43) Date of publication of application: **08.12.2010**
(21) Application number: 09161038.6
(22) Date of filing: 25.05.2009
(51) Int. Cl.: C12Q 1/68

(54) **miRNA biomarkers for the diagnosis of duchenne muscular dystrophy progression, for monitoring therapeutic interventions, and as therapeutics**

(71) Applicant: Universita'Degli Studi di Roma "La Sapienza", 00185 Roma (IT)
(72) Inventor: Bozzoni, Irene, 00185 Rome (IT); Martone, Julie, 00185 Rome (IT); Cacchiarelli, Davide, 00185 Rome (IT); Girardi, Erika, 00185 Rome (IT)
(74) Representative: Capasso, Olga

(57) **Abstract**

The invention refers to diagnosis and therapy of muscle degenerative disorders, as Duchcnnc Muscular Dystrophy (DMD by means of a class of specific miRNAs. In particular the invention refers to a method for the diagnosis of a muscle disorder and damage leading to muscle fiber degeneration, inflammation and fibrosis or for monitoring the progress of therapeutic treatments on subjects affected by said disorder consisting in detecting in a biological sample at least two of dystromiR molecules, each of the two dystromiR molecules belonging to one of the following groups: a) muscle regeneration (miR-206, miR-34), b) muscle differentiation (mix-1, miR-133), c) inflammatory (miR-223, miR-21) and d) tissue integrity (miR-29, miR-30).

SEQUENCE LISTING

<110>  Università degli Studi di Roma "La Sapienza"

<120>  miRNA BIOMARKERS FOR THE DIAGNOSIS OF DUCHENNE MUSCULAR DYSTROPHY
        PROGRESSION, FOR MONITORING THERAPEUTIC INTERVENTIONS, AND AS
       THERAPEUTICS

<130>  BE106059

<160>  22

<170>  PatentIn version 3.5

<210>  1
<211>  22
<212>  RNA
<213>  Homo sapiens

<400>  1
uggaauguaa agaaguaugu au                                              22


<210>  2
<211>  22
<212>  RNA
<213>  Homo sapiens

<400>  2
uggaauguaa ggaagugugu gg                                              22


<210>  3
<211>  22
<212>  RNA
<213>  Homo sapiens

<400>  3
ugucaguuug ucaaauaccc ca                                              22


<210>  4
<211>  22
<212>  RNA
<213>  Homo sapiens

<400>  4
ugucaguuug ucaaauaccc ca                                              22


<210>  5
<211>  22
<212>  RNA
<213>  Homo sapiens

<400>  5
uagcaccauc ugaaaucggu ua                                              22


<210>  6
<211>  23
<212>  RNA
<213>  Homo sapiens

<400>  6
uagcaccauu ugaaaucagu guu                                             23


<210>  7

<211>  22
<212>  RNA
<213>  Homo sapiens

<400>  7
uagcaccauu ugaaaucggu ua                                                22


<210>  8
<211>  21
<212>  RNA
<213>  Homo sapiens

<400>  8
aggcaagaug cuggcauagc u                                                 21


<210>  9
<211>  22
<212>  RNA
<213>  Homo sapiens

<400>  9
uggcaguguc uuagcugguu gu                                                22


<210>  10
<211>  22
<212>  RNA
<213>  Homo sapiens

<400>  10
uuuggucccc uucaaccagc ug                                                22


<210>  11
<211>  22
<212>  RNA
<213>  Homo sapiens

<400>  11
uuuggucccc uucaaccagc ua                                                22


<210>  12
<211>  23
<212>  RNA
<213>  Homo sapiens

<400>  12
uguaaacauc cuacacucuc agc                                               23


<210>  13
<211>  24
<212>  DNA
<213>  Artificial Sequence

<220>
<223>  primer

<400>  13
cagtgtccat tcatctccta cagc                                              24


<210>  14
<211>  24
<212>  DNA
<213>  Artificial Sequence

<220>
<223>  primer

<400>  14
gcttggaata tgctgacagg ctgc                                              24


<210>  15
<211>  24
<212>  DNA
<213>  Artificial Sequence

<220>
<223>  primer

<400>  15
caaagcagtg ttgggaccac agga                                              24


<210>  16
<211>  23
<212>  DNA
<213>  Artificial Sequence

<220>
<223>  primer

<400>  16
actcccaggc aaacagactc tct                                               23


<210>  17
<211>  24
<212>  DNA
<213>  Artificial Sequence

<220>
<223>  primer

<400>  17
ctgtggagac aggtagtgaa gctc                                              24


<210>  18
<211>  24
<212>  DNA
<213>  Artificial Sequence

<220>
<223>  primer

<400>  18
tgaactggaa gggagccaac atgg                                              24


<210>  19
<211>  24
<212>  DNA
<213>  Artificial Sequence

<220>
<223>  primer

<400>  19
cgtcgatgaa gaacgcagct agct                                              24


<210>  20

<211>  24
<212>  DNA
<213>  Artificial Sequence

<220>
<223>  primer

<400>  20
gtgtcgatga tcaatgtgtc ctgc                                              24


<210>  21
<211>  24
<212>  DNA
<213>  Artificial Sequence

<220>
<223>  primer

<400>  21
gtgattatct gggaatcctc tggg                                              24


<210>  22
<211>  24
<212>  DNA
<213>  Artificial Sequence

<220>
<223>  primer

<400>  22
ccggaactct tatctgacta ggtg                                              24

## Description

### Field of the invention

The invention refers to diagnosis and therapy of muscle degenerative disorders, as Duchenne Muscular Dystrophy (DMD by means of a class of specific miRNAs.

### Background

In Duchenne Muscular Dystrophy, the absence of dystrophin leads to a dramatic decrease of the Dystrophin-Associated Protein Complex (DAPC) required to connect intracellular actin microfilaments to the extracellular matrix (Matsumura et al., 1994; Ervasti et al., 2008). As a consequence, the muscle fibers become more sensitive to mechanical damage leading to muscle degeneration, chronic inflammatory response and increase in fibrosis, all of which exacerbate the dystrophic phenotype.

Making use of the exon skipping strategy (Denti et al., 2006) for the rescue of dystrophin synthesis in Duchenne mice *(mdx)*, the authors discovered that this protein, besides its structural function, is also able to alter the expression pattern of a specific subset of miRNA genes relevant for muscle differentiation (Chen et al., 2006) and proper tissue morphology (Van Rooij et al., 2008).

The authors show that this effect is mediated by dystrophin-dependent nNOS re-localization to the membrane, activation of the NO signalling and HDAC2 release from the chromatin (Wehling et al., 2001) of these genes. While in the absence of dystrophin, such as in human DMD myoblasts and mdx mice, these miRNAs are repressed, in conditions of dystrophin synthesis they are transcriptionally active.

These results also indicate that the morpho-functional benefit observed in dystrophin-rescued *mdx* animals (Denti et al., 2006) is not simply due to restored membrane integrity but rather to the activation of specific genetic programmes required for proper muscle structure.

The link between miRNA expression, NO pathway and dystrophin is supported by the observation that beside this structural role, the Dystrophin-Associated Protein Complex is required for the localization and activation of the sarcolemmal neuronal Nitric Oxide Synthase (nNOS): impaired NO production in dystrophic muscles was demonstrated to be due to the disruption of the association between the sarcolemmal neuronal Nitric Oxide Synthase (nNOS) and DAPC (Brenman et al., 1995).

Nitric Oxide has recently emerged as a crucial key player mediating epigenetic changes through the direct control of Histone deacetylases (HDACs). In neurons and skeletal muscles, a NO-dependent S-nitrosylation of HDAC2 resulted in its release from the target DNA sequences with consequent changes of histone modification and activation of genes for neuronal development (Nott et al., 2008). On the contrary, in dystrophin-deficient *mdx* mice, defective for the NO-pathway, the activity of HDAC2 was specifically increased (Colussi C. et al., 2008). At variance with class I, class II histone deacetylase activity was instead enhanced by NO, by increased HDAC4 and 5 nuclear translocation (Illi et al., 2008).

The relevance of NO and HDAC activities in the Duchenne pathology, is supported on one side from the evidence that Nitric Oxide release in animals ameliorated the dystrophic phenotype (Brunelli et al., 2007) and on the other that deacetylase inhibitors conferred strong morpho-functional benefit to dystrofic muscle fibers. In the latter case changes in gene expression patterns, such as the transcriptional activation of follistatin, the myostatin antagonist, were described (Minetti et al., 2006). Therefore, a direct link between dystrophin and gene reprogramming through alteration of the epigenetic signature has been suggested.

### Description of the invention

miRNAs are molecules known to play crucial functions in the differentiation commitment of several cell types and to be involved in many patho-physiological processes. In this work, authors identified a specific signature of miRNAs that correlated with the DMD pathology. They found a different miRNA expression profile between wild type and Duchenne cells, both in human DMD and in *mdx* mice. Furthermore, miRNA profile analysis in *cells* in which dystrophin has been rescued through the exon skipping approach indicated the existence of a specific class of miRNAs that are directly controlled by dystrophin: some of them being important for muscle differentiation and regeneration. A different group of miRNAs was found to change as a consequence of the benefit of the therapeutic treatment after dystrophin rescue; this class includes miRNAs diagnostic of the inflammatory and fibrotic processes.

The authors propose that the observed differences in the expression levels of both classes of miRNAs (dystromiR) can be utilized as biomarkers in order to evaluate the severity/progression of the disease in human patients as well as for measuring the outcomes of therapeutic interventions. In consideration of their link with muscle degeneration these miRNAs can be used as diagnostic tools of other types of muscular disorders in which fiber degeneration occurs with subsequent side effects such as inflammation and fibrosis. Moreover, authors were able to measure alterations of the dystromiR profile directly in serum samples and muscle biopsies in a quantitative and rapid way.

Finally, they found that specific miRNAs (dystromiR) can be utilized as new therapeutic tools for the treatment of DMD: their administration to Duchenne muscles was shown to confer strong morphological benefit.

Relevant dystromiR are:
- **miR-31.** It increases by 50-70 fold in dystrophic muscles; however, its level is not modified by rescue of dystrophin synthesis. It is very useful for studying disease progression. Since it targets dystrophin mRNA, its depletion can increase dystrophin translation with potential therapeutic benefits.
- **miR-223, miR-21.** They are expressed in inflammatory cells and inflammation is known to be very strong in Duchenne muscles. Upon dystrophin rescue the values are corrected to almost wild type levels due to the beneficial effect of dystrophin rescue on tissue integrity.
- **miR-29, miR-30** They are down-regulated in *mdx* muscles. Members of the miR-29 and miR-30 families have been recently shown to target mRNAs coding for crucial factors involved in muscle damage (Haslett et al., 2002). Among them, collagens and elastin, integral components of the extracellular matrix, known to increase in *mdx* animals where they contribute to the massive fibrosis typical of Duchenne muscles. Upon dystrophin rescue their levels are restored to almost wild type levels. Delivery of miR-29 to *mdx* muscles recovers wild type levels of collagen and elastin mRNAs and reduces fibrosis. Therefore, miR-29 and/or similarly miR-30 represents new potential therapeutic tools for improving several features of dystrophic muscles such as fibrosis. They can be utilized in conjunction with other therapies (i.e. exon skipping) to improve the effectiveness of the therapeutic treatment. They can be administered either as synthetic molecules or as gene constructs with muscle specific promoters in the backbone of viral constructs. The delivery route can be either by local or systemic administration.
- **miR-206 and miR-34.** They correlate with the amount of regenerating fibers. They increase in dystrophic muscles paralleling muscle damage. They further increase after exon skipping when muscle regeneration is strongly enhanced. miR-206 levels increase in the serum of dystrophic mice .
- **miR-1 and miR-133** They are markers of muscle differentiation. Their accumulation decreases in dystrophic muscles while it is restored upon dystrophin rescue. miR-1 levels increase in the serum of dystrophic mice.

Therefore it is an object of the instant invention a method for the diagnosis of a muscle disorder and damage leading to muscle fiber degeneration, inflammation and fibrosis or for monitoring the progress of therapeutic treatments on subjects affected by said disorder consisting in detecting in a biological sample at least two of dystromiR molecules, each of the two dystromiR molecules belonging to one of the following groups: a) muscle regeneration (miR-206, miR-34), b) muscle differentiation (miR-1, miR-133), c) inflammatory (miR-223, miR-21) and d) tissue integrity (miR-29, miR-30). In a preferred aspect the muscle disorder is the Duchenne Muscular Dystrophy. In this case each of the two dystromiR molecules belongs preferably to one of the following groups: a) muscle regeneration (miR-206, miR-34), b) muscle differentiation (miR-1, miR-133), c) inflammatory (miR-223, miR-21) and d) tissue integrity (miR-29, miR-30); Duchenne specific (miR-31), more preferably the at least two of dystromiR molecules are selected in the following group: miR-31, miR-206, miR-1, miR-223 and miR-29. The detection of such molecules shall be compared with appropriate controls to monitor the increase/decrease of such molecules in the analyte sample.

In an aspect of the invention the method is performed on a muscle bioptic sample, alternatively on a serum or a blood sample.

Any way of detection of the at least two of dystromiR molecules in a biological sample is within the instant invention. Preferred methods involve reverse specific amplification and real time detection of amplified products.

It is another object of the invention a kit to perform the method as above described.

It is another object of the invention a dystromiR molecule belonging to the group of miR-29 and/or miR-30 for use as therapeutic medicament of Duchenne Muscular Dystrophy or of pathologies associated to mutations of DAPC components, such as Girdle Limb dystrophy, or as coadiuvant of other therapeutic strategies, such as exon skipping.

It is another object of the invention a pharmaceutical composition for local and/or systemic administration comprising the dystromiR molecule or a vector encoding the dystromiR molecule according to claim 9.

It is another object of the invention an agent able to functionally inactivate the dystromiR-31 molecule, preferably as coadiuvant of other therapeutic strategies, such as exon skipping.

### Detailed description of the invention

### Figure legends.

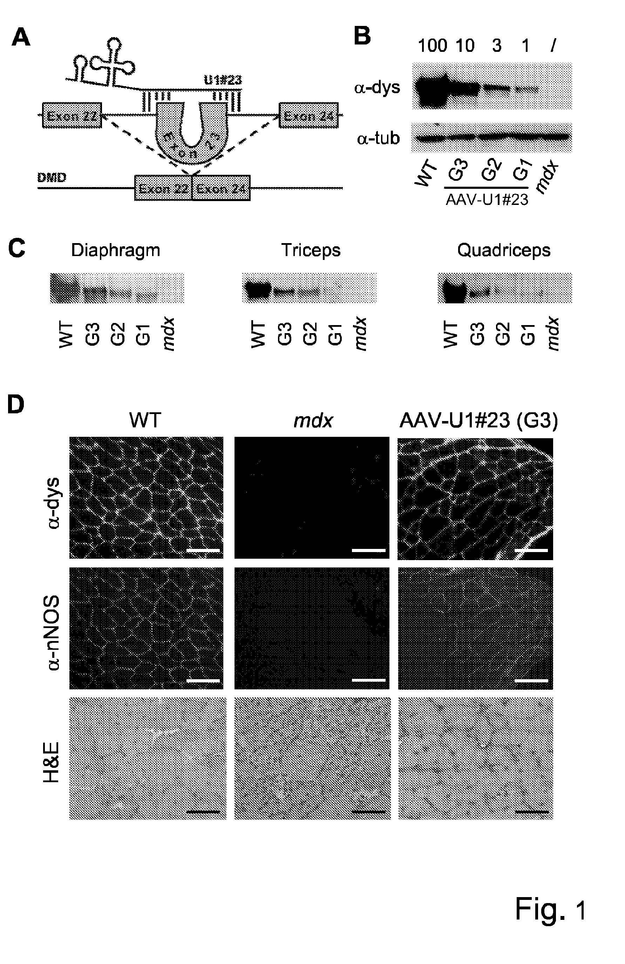

**Figure 1****. Analysis of dystrophin and nNOS rescue upon exon skipping treatment.** (A) Schematic representation of the exon skipping strategy. (B)Western blot with anti-dystrophin (αDys) and α-tubulin (α-tub) antibodies on 50 µg of proteins from gastrocnemius of wild type (WT) animals and 50 µg of *mdx,* G1, G2 and G3 animals 4 weeks after systemic injection of AAV-U1#23. (C)Western blot with anti-dystrophin (αDys) antibody on 50 µg of proteins from triceps, diaphragm and vastus from *mdx,* G1, G2, G3 and wild type (WT) animals 4 weeks after systemic injection of AAV-U1#23. (C) Muscles from wild type (WT), control (*mdx*) and AAV-U1#23 injected *mdx* mice were sliced and analyzed by immunofluorescence with anti-dystrophin and anti-nNOS antibodies. The morphological state was analyzed by Hematoxilin/Eosin (H&E) staining. Original magnification, X20. Scale bar is 100 µm.

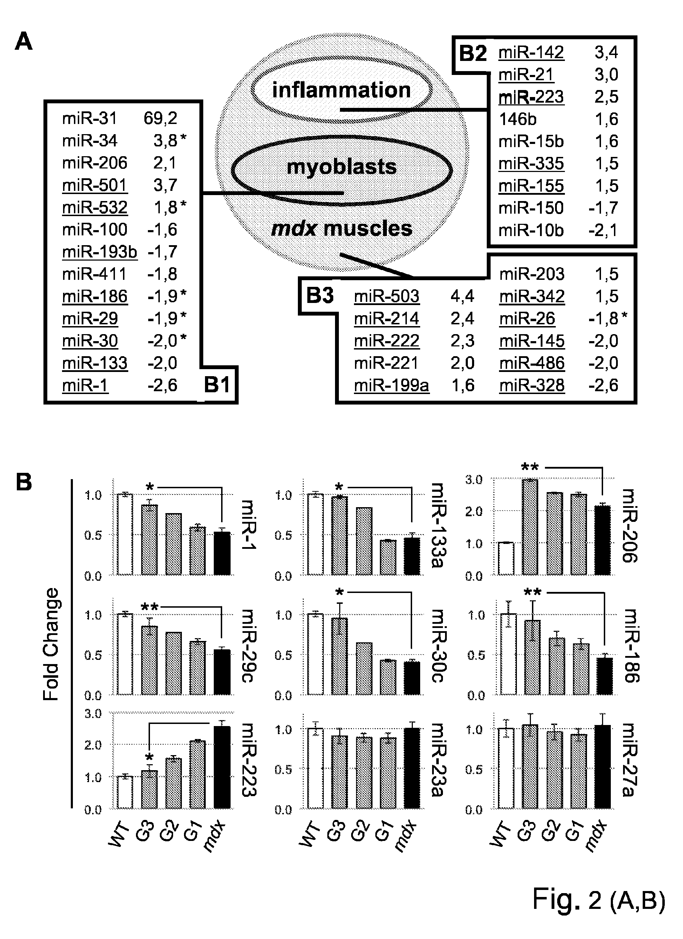

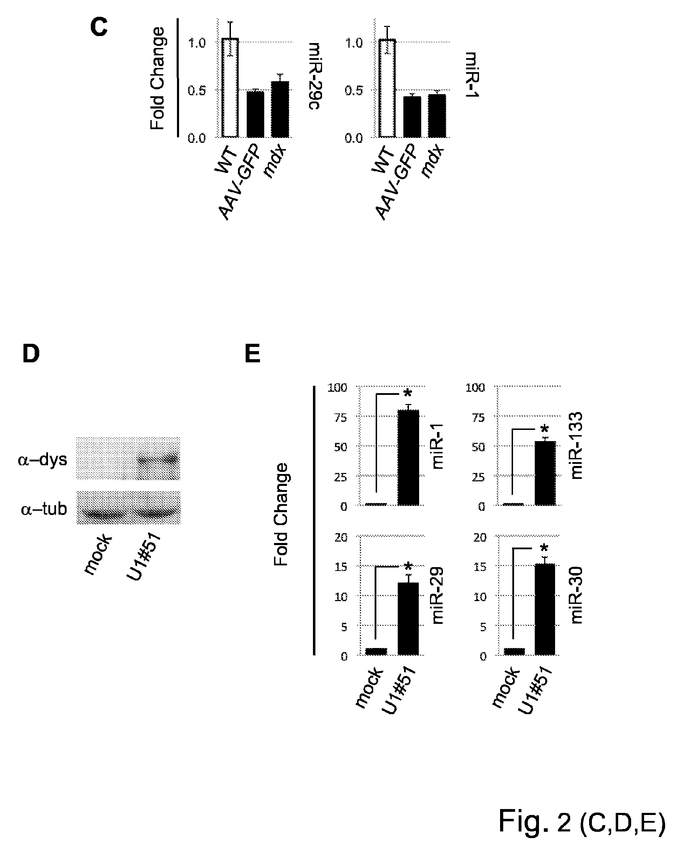

**Figure 2** **- Analysis of miRNA expression.** (A) A list of differentially expressed miRNAs (at least 1,5 fold variation), in WT and *mdx* gastrocnemius, was derived from the values of real-time based low density arrays (Table 1). Aware of tissue heterogenity, the diagram shows miRNA classification into three groups: Box1 (B1), Box2 (B2), Box3 (B3). For each miRNA the fold change value between WT and *mdx* is shown. Dots indicate the mean expression of miRNAs belonging to the same family; miRNAs, rescued towards WT levels in exon skipping-treated mice, are underlined. (B) Histograms show miRNA relative expression in gastrocnemius from WT (white bars), G1-G3 (grey bars) and *mdx* (black bars) mice, measured by qRT-PCR. The invariant miR-23a and miR-27a were used as controls. Fold changes are normalized to snoRNA55 and shown with respect to WT set to a value of 1. (C) AAV administrations do not affect miRNA expression. *mdx* animals were tail-vein injected with a AAV-vector encoding only for GFP (AAV-GFP) and sacrificed after 4 weeks in parallel with wild type (WT) and untreated *mdx* animals. miR-1 and miR-29 levels were measured by qRT-PCR on RNA from gastrocnemius. Fold changes are shown with respect to WT, set to a value of 1 and normalized to snoRNA55. (D) The human DMD deletion of exons 48-50 of the dystrophin gene produces an out of frame mRNA. Skipping of exon 51 would produce an in frame mRNA and the synthesis of a shorter but functional dystrophin protein. Human cultured DMD myoblasts carrying the exon 48-50 deletion were infected with a lentiviral construct carrying antisense sequences against the splice junctions of exon 51 (De Angelis et al. 2002). Cells were maintained in DM medium for 7 days. Western blot with anti-dystrophin (α-dys) antibodies on 50 µg of proteins from mock infected (mock) and antisense-treated (U1#51) DMD cells indicates the efficient recovery of dystrophin synthesis. Anti-tubulin antibodies (α-tub) were used as control. (E) qRT-PCR on the indicated miRNAs in control (mock) and antisense-treated (U1#51) DMD cells. U6 snRNA was used as endogenous control. Fold changes are shown with respect to mock-infected DMD cells, set to a value of 1. One asterisk (p<0.01), two asterisks (p<0.05).

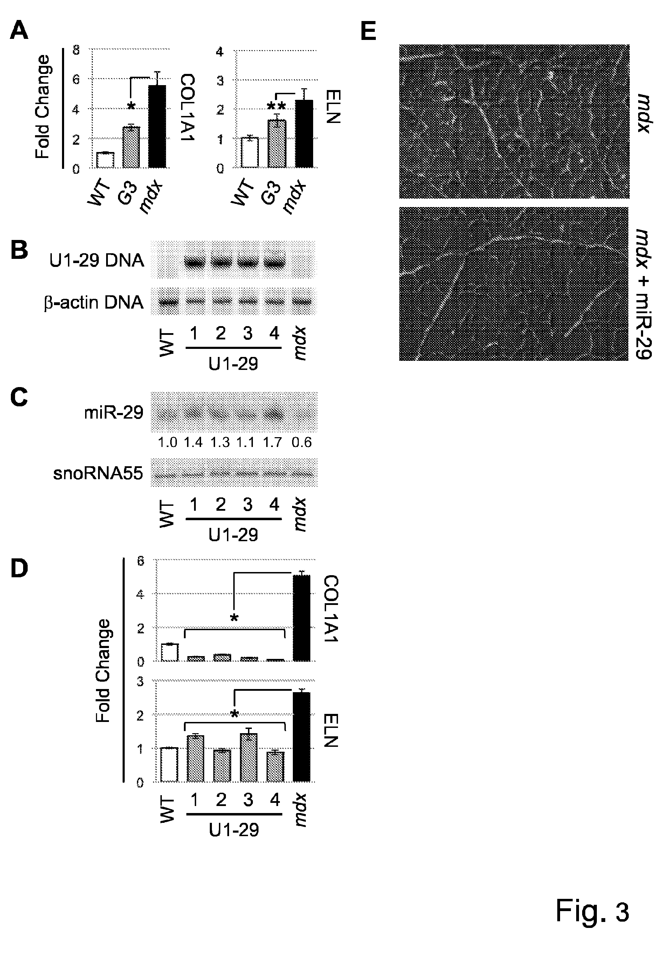

**Figure 3** **- Analysis of miR-29 targets.** (A) qRT-PCR for the collagen (COL1A1) and elastin (ELN) mRNAs on total RNA from the gastrocnemius of WT (white bars), G3 (grey bars) and *mdx* (black bars) mice. Fold changes are normalized to HPRT1 mRNA and shown with respect to WT set to a value of 1. In panels B-E the gastrocnemius of *mdx* animals were electroporated with a miR-29a/b expression construct (U1-29) and sacrificed after 25 days. (B) PCR analysis on DNA from WT, *mdx* and U1-29 electroporated (1-4) *mdx.* Primers specific for the U1-29 construct and for the β-actin gene were utilized. (C) Northern blot for miR-29a and control snoRNA55. Normalized miR-29a relative expression level is indicated below each lane. (D) qRT-PCR on COL1A1 and ELN mRNAs. One asterisk (p<0.01), two asterisks (p<0.05). (E) The morphological state of muscles from control (*mdx*) and U1-29 electroporated *mdx* mice was analyzed by Trichromic staining. Original magnification, X4.

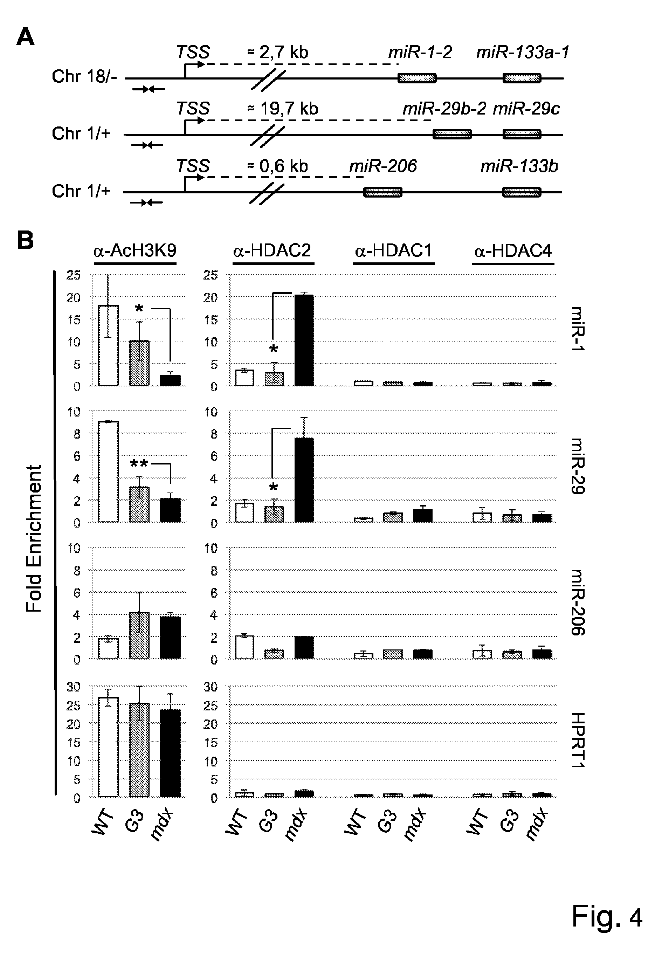

**Figure 4** **- Chromatin immunoprecipitation analysis on microRNA promoters.** (A) Schematic representations of the coding and promoter regions of miR-1-2/133a-1, miR-29b-2/29c and miR-206/133b genes. The transcriptional start site (TSS) and the primers utilized for PCR amplifications are indicated. (B) ChIP analyses were performed on chromatin from gastrocnemius of WT (white bars), G3 (grey bars) and *mdx* (black bars) mice with antibodies against AcH3K9, HDAC1, HDAC2 and HDAC4. The immunoprecipitated DNA samples were amplified with specific primers spanning miR-1, miR-29 and miR-206 regulatory regions. The Hprt1 promoter region was analyzed as control. Oligonucleotides for 5,8s rDNA were used as internal control by coamplification with each of the specific primers. Data are mean ± s.d. from three independent experiments. Results are expressed as fold enrichment of DNA immunoprecipitated samples relative to INPUT chromatin.

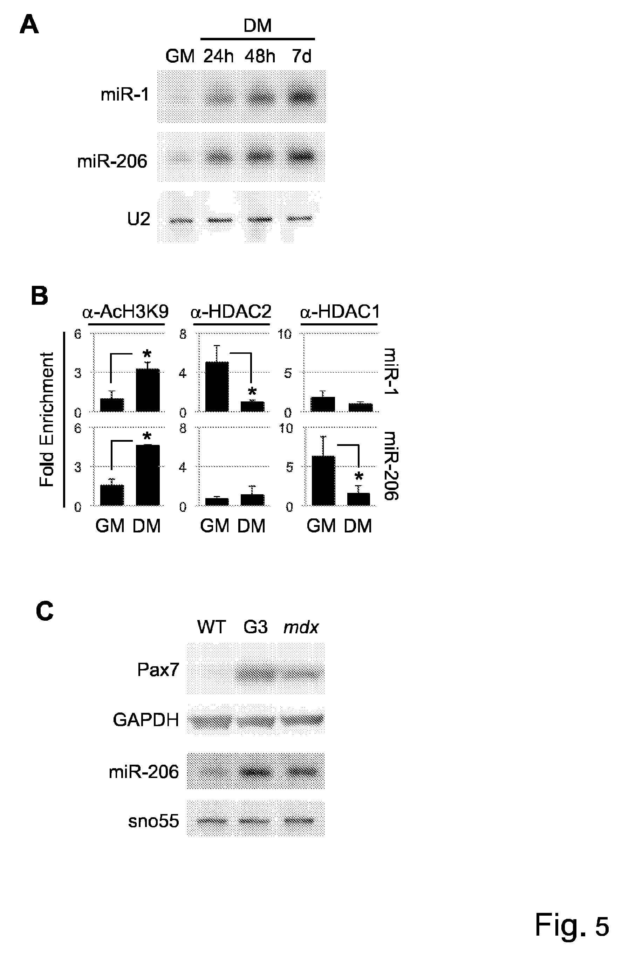

**Figure 5** **- Analysis of miR-206 and Pax7 expression.** (A) miR-206 and miR-1 expression was analyzed by Northern blot in C₂7 cells maintained in growth medium (GM) or in differentiation medium (DM) for the indicated times. U2 snRNA was utilized as loading control. (B) ChIP analysis with antibodies against AcH3K9, HDAC1 and HDAC2 was performed on miR-206 and miR-1-2 promoters in GM and DM (7 days). (C) Western analysis of Pax7 (upper panel) and Northern for miR-206 (lower panel) in the gastrocnemius of WT, G3 and *mdx* animals. Signals were normalized for GAPDH and snoRNA55, respectively.

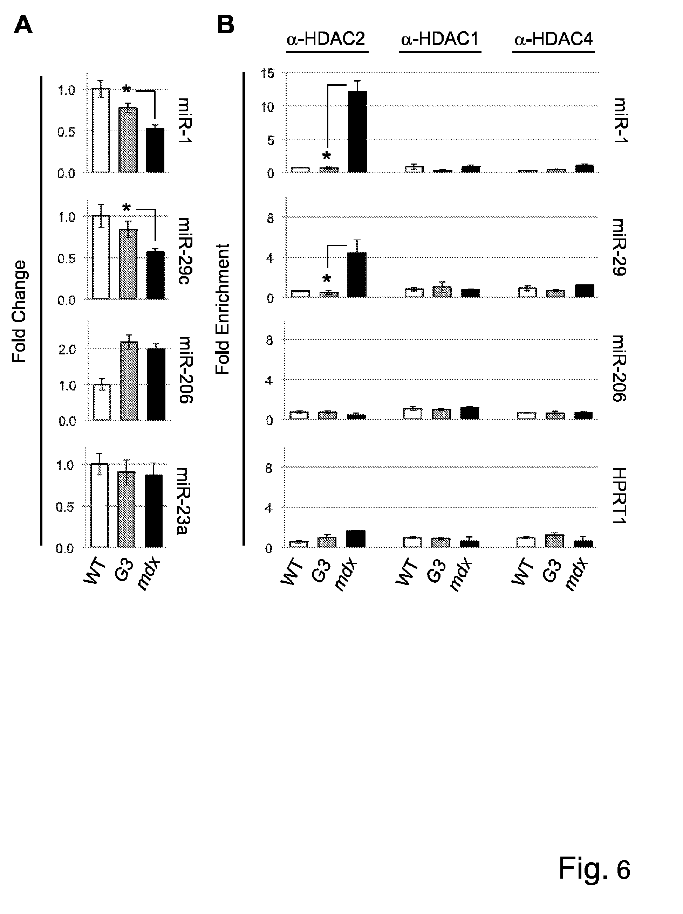

**Figure 6** **- Responsiveness of miRNA expression to the NO signalling.** (A) qRT-PCR on miR-1, miR-29, miR-206 in WT (white bars), G3 (grey bars) and *mdx* (black bars) mice treated for 4 hours with nitroglycerin (NTG). miR-23, is utilized as an internal invariant control miRNA. (B) α-HDAC2 ChIP analyses were performed on chromatin from gastrocnemius of WT, *mdx* and NTG-treated *mdx* mice. The immunoprecipitated DNA samples were amplified with specific primers spanning miR-1, miR-29 and miR-206 regulatory regions (see Fig. 4). The Hprt1 promoter region was utilized as control. Oligonucleotides for 5,8s rDNA were used as internal control by co-amplification with each one of the specific primers. Data are mean ± s.d. from three independent experiments. Results are expressed as fold enrichment of DNA immunoprecipitated samples relative to INPUT chromatin. No antibody was used as control.

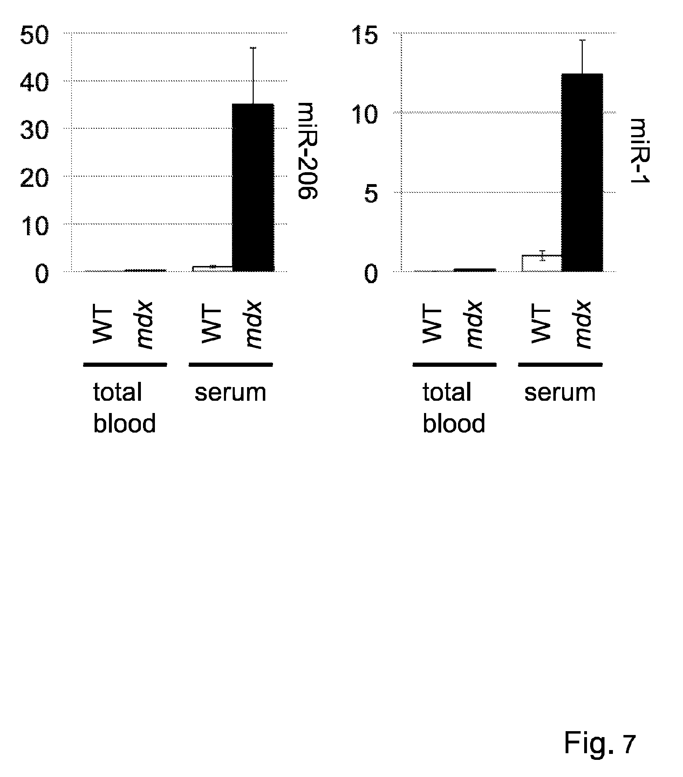

**Figure 7** **- miRNA expression in blood and serum**. Histograms show miR-206 and miR-1 relative expression in total blood and serum from WT (white bars) and *mdx* (black bars) mice, measured by qRT-PCR. Fold changes are normalized to snoRNA55 and shown with respect to WT serum levels set to a value of 1.

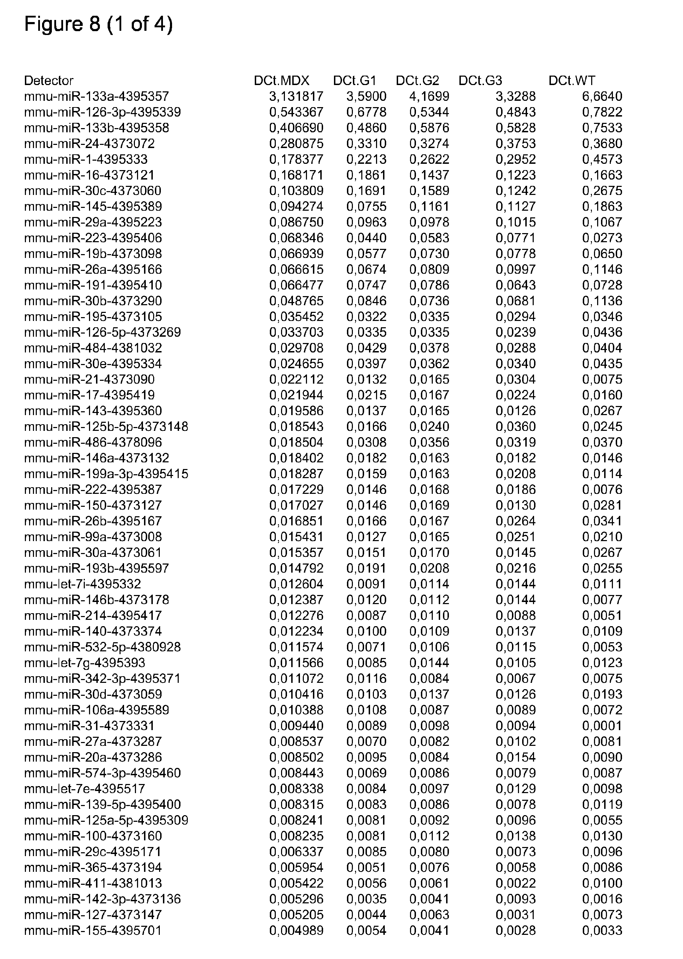

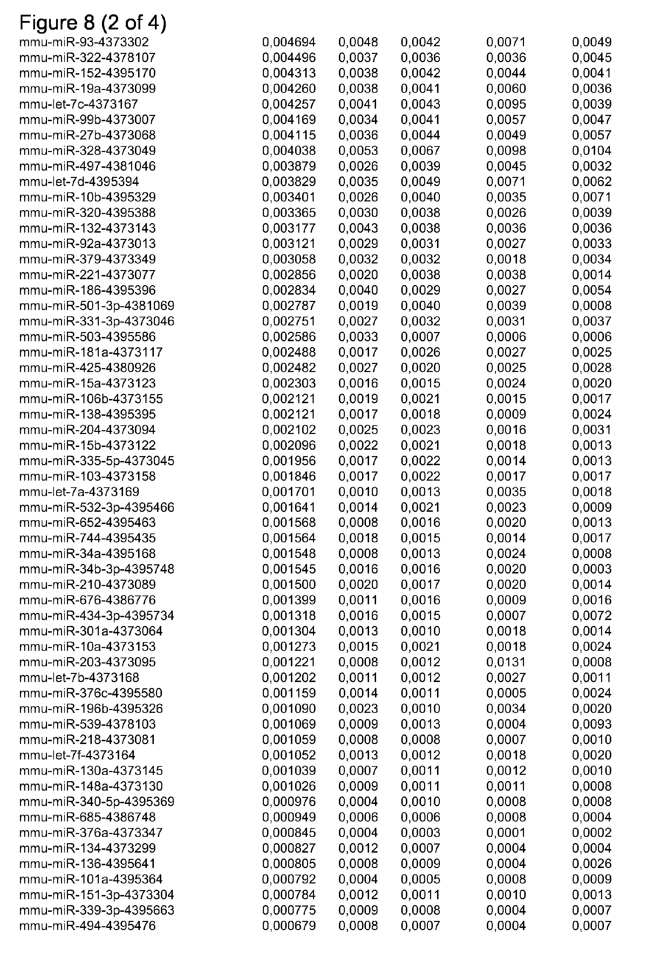

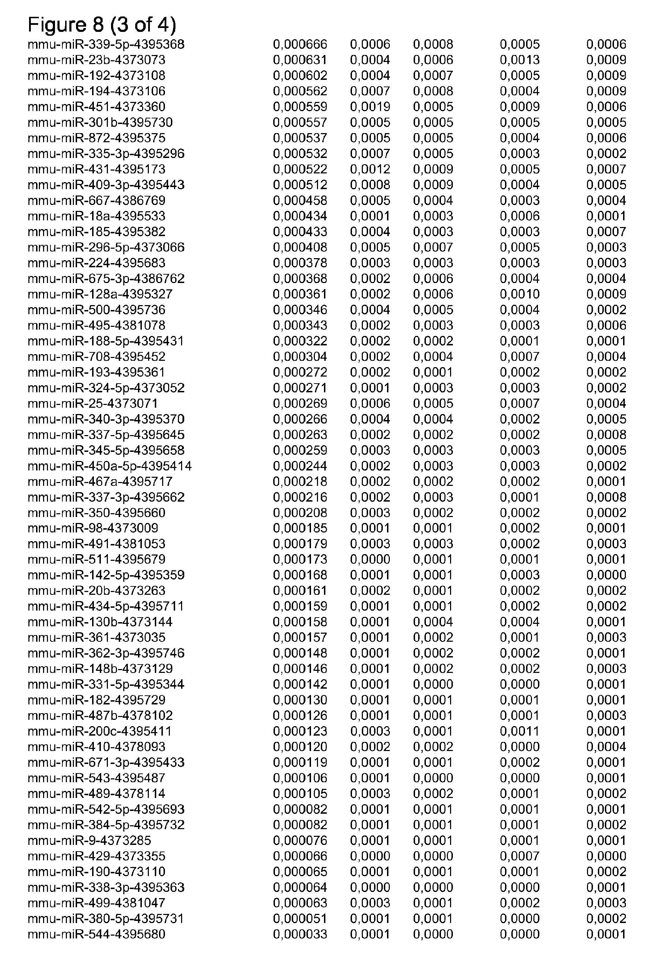

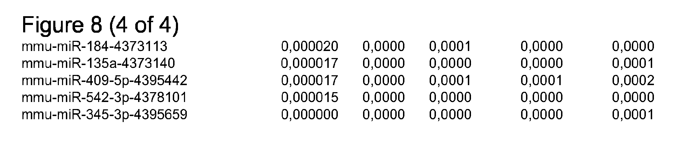

Figure 8 **-** A real time PCR-based approach (Applied Biosystems Taqman® Megaplex Array) was applied to total RNA derived from the gastrocnemius of a pool of three different individuals of WT (column DCt.WT), *mdx* (column DCt.MDX) and three independent AAV-U1#23 treated mice (columns DCt.G1, DCt.G2, DCt.G3). miRNA Raw ΔCt values are indicated and ranked from the highest to the lowest expression level in *mdx.*

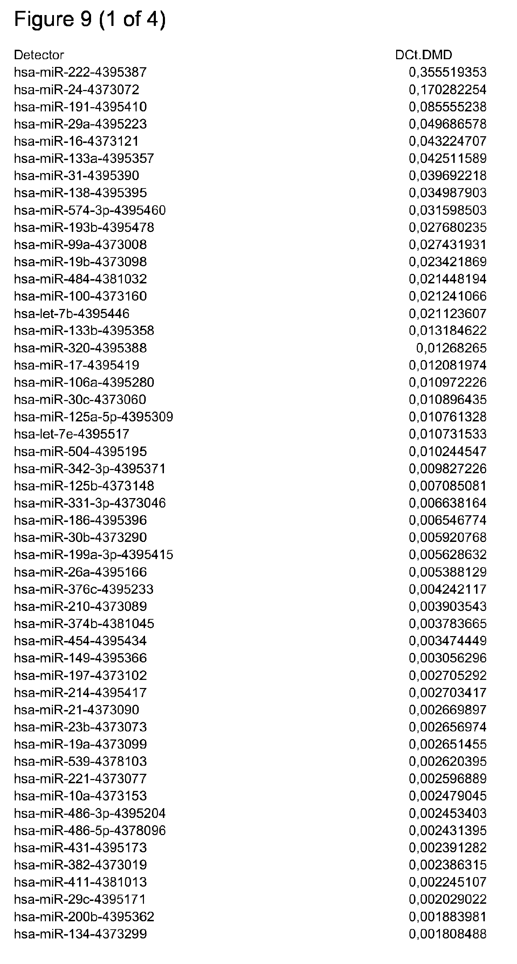

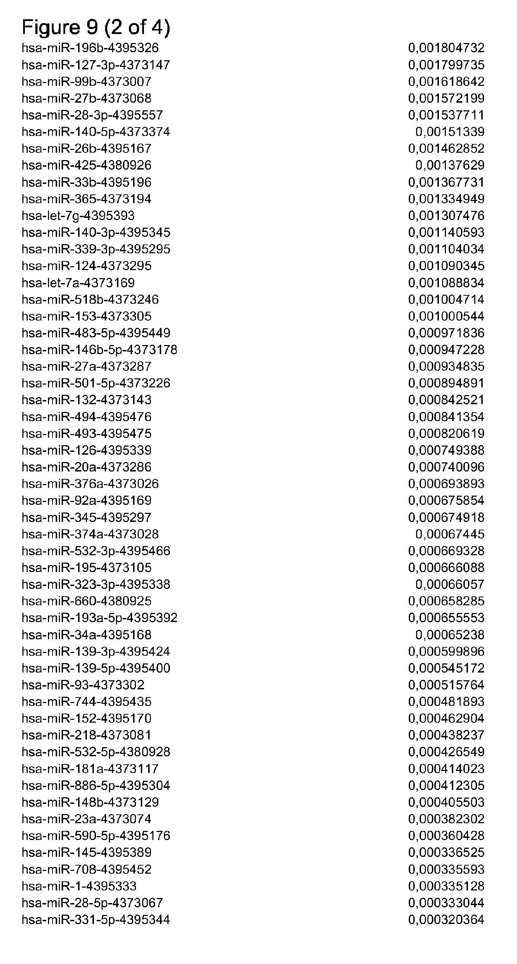

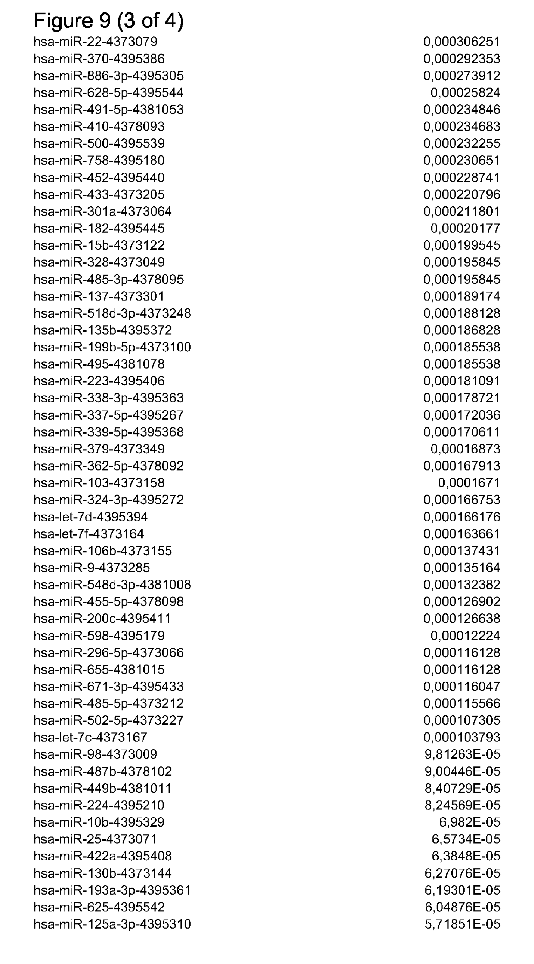

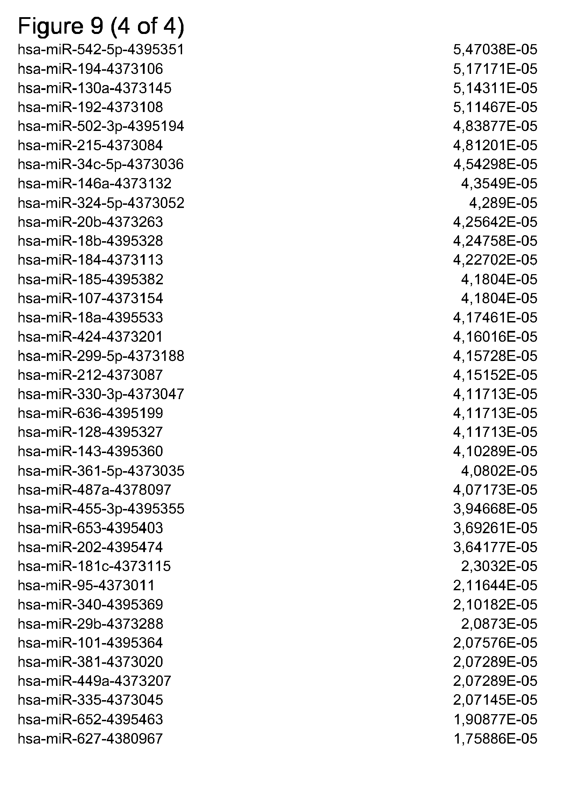

Figure 9 **-** A real time PCR-based approach (Applied Biosystems Taqman® Megaplex Array) was applied to total RNA derived from DMD myoblasts induced to differentiate. Raw ΔCt values are indicated and ranked from the highest to the lowest (column DCt.DMD).

### Materials and Methods

### Sequence of miRNAs under analysis

Mature miRNAs as below show perfect sequence conservation between human and mouse. The mature sequence of the miRNA not listed can be found on the public database of miRNAs (www.mirbase.org).
miR-1: uggaauguaaagaaguauguau (SEQ ID No. 1) (the same mature miRNA sequence derives from the two miRNA genes (miR-1-1 and miR-1-2).
miR-206: uggaauguaaggaagugugugg (SEQ ID No. 2)
miR-223: ugucaguuugucaaauacccca (SEQ ID No. 3)
miR-21: ugucaguuugucaaauacccca (SEQ ID No. 4)
miR-29a: uagcaccaucugaaaucgguua (SEQ ID No. 5)
miR-29b1/2: uagcaccauuugaaaucaguguu (SEQ ID No. 6)
miR-29c: uagcaccauuugaaaucgguua (SEQ ID No. 7)

The miR-29 family includes miR-29a miR-29b1/2 and miR-29c (each produced from a specific locus). When miR-29 expression levels are indicated, the reported values are the mean result of all the three mature miRNAs. Also in this case the same mature miRNA sequence can derive from different genomic locations (miR-29b-1 and miR-29b-2 genes).
miR-31: aggcaagaugcuggcauagcu (SEQ ID No. 8)
miR-34a: uggcagugucuuagcugguugu (SEQ ID No. 9)
miR-133a1/2: uuugguccccuucaaccagcug (SEQ ID No. 10)
miR-133b: uuugguccccuucaaccagcua (SEQ ID No. 11)

The miR-133 family includes miR-133a1/2 and miR-133b (each produced from a specific locus). When miR-133 expression levels are indicated, the reported values are the mean result of the two mature miRNAs. Also in this case the same mature miRNA sequence can derive from different genomic locations (miR-133a-1 and miR-133a-2 genes).
miR-30c1/2: uguaaacauccuacacucucagc (SEQ ID No. 12)

### Animal treatments.

6 weeks-old *mdx* mice were tail vein injected with 0.5-1x10¹² genome copies of the AAV-U1#23 virus as described in Denti et al.(2006) and sacrificed after 4 weeks. Electroporation was performed on 6 week-old *mdx* as already described (Donà et al., 2003) with an expression cassette carrying the 29a/b coding region under the control of the U1 snRNA promoter (U1-29a/b). Nitroglycerin treatment was performed on 18 week-old *mdx* mice locally injected in gastrocnemius with 0,3 mg/kg of nitroglycerin. *mdx* control mice were injected with physiological solution. Animals were sacrificed after 5 hours.

**RNA Preparation and Analysis**. Total RNA was prepared from liquid nitrogen powdered tissue homogenized in TRIzol reagent (Invitrogen). RNA from blood and serum samples was extracted using TRIreagent BD (Sigma). miRNA profiling was performed using Applied Biosystems TaqMan Megaplex Low Density Array. (See supplementary methods online), while analysis of individual miRNAs was performed using specific TaqMan microRNA assay (Applied Biosystems). Relative quantification was performed using sno55 as endogenous control for murine samples and U6 for human samples. Data were expressed as means ± SEM, unless otherwise stated. Statistical significance of the differences between means was assessed by t-test (nonparametric). A probability of <5% was considered significant.

### Protein and Immunofluorescence Analyses.

Western blot on total extract and in situ analyses on 7 µm-thick gastrocnemius cryosections were performed as described in Denti et al (2006). The NCL-DYS2 (Novocastra) 1:12.5; nNOS (Santa Cruz Biotechnology) 1:10. monoclonal antibodies were used.

### Chromatin immunoprecipitation assays.

ChIP analysis was performed as described in suppl. methods online with the following antibodies: HDAC2 (Santa Cruz Biotechnology), HDAC1, HDAC4, acetylated H3-Lys9 (Upstate Biotechnology).

### miRNA profiling and data analysis.

To synthesize single-stranded cDNA, 700 ng of total RNA were reverse transcribed using the miRNA reverse transcription kit in combination with the stem-loop Megaplex RT primers pool A (Applied Biosystem), allowing the simultaneous reverse transcription of 335 miRNAs, 5 species-specific endogenous controls and one negative control. From the cDNA samples, 335 small RNAs were profiled using an Applied Biosystems TaqMan Low Density Array. Since instrument and liquid handling variations were shown to be minimal, no PCR replicates were measured according to manufacturer specifications. PCR amplification reactions were carried out in a total volume of 1,5 microL. Raw Ct values were calculated using the SDS software V.2.3 using automatic baseline settings and a threshold of 0,2. Data were subsequently analyzed through StatMiner platform according to the manufacturer pipeline. StatMiner Genorm algorithm was applied to determine the best invariant endogenous controls on a list of 5.

### Oligonucleotides used in ChIP assays:

206 fw:cagtgtccattcatctcctacagc (SEQ ID No. 13)
206 rw:gcttggaatatgctgacaggctgc (SEQ ID No. 14)
1-2 fw:caaagcagtgttgggaccacagga (SEQ ID No. 15)
1-2 rw:actcccaggcaaacagactctct (SEQ ID No. 16)
29 fw:ctgtggagacaggtagtgaagctc (SEQ ID No. 17)
29 rw:tgaactggaagggagccaacatgg (SEQ ID No. 18)
rma fw:cgtcgatgaagaacgcagctagct (SEQ ID No. 19)
rma rw:gtgtcgatgatcaatgtgtcctgc (SEQ ID No. 20)
hprt fw:gtgattatctgggaatcctctggg (SEQ ID No. 21)
hprt rw:ccggaactcttatctgactaggtg (SEQ ID No. 22)

### Chromatin immunoprecipitation (ChIP)-quantitative PCR (qPCR) assay.

Chromatin immunoprecipitation (ChIP) assay. Gastrocnemius (0,1-0,2 g) were dissected and incubated in 1,8% formaldehyde for 20 minutes at 22°C (after 5 minutes they were cut in pieces of 1-3 mm3). C27 myoblasts (1x106 cells/ml) were instead incubated in 1% formaldehyde for 10 minutes at 25°C. The reactions were stopped by glycine (0,125 M) for 5 minutes at RT. After washing with cold PBS 1X, samples were collected by centrifugation at 4 C° at 3000 rpm, resuspended in 5 volumes of lysis buffer (SDS 1%, EDTA 10 mM TRIS-HCl 50 mM pH 8) and sonicated. The average DNA fragment length was 400-1000 bp. Samples were then centrifuged at 10,000 rpm for 10minutes at 4°C. Supernatant was diluted with ChIP dilution buffer (SDS 0,01%, Triton X-100 1,1%, EDTA 1,2 mM, Tris HCl 16,7 mM pH 8, NaCl 167 mM) and pre-cleared with 50 µl of Protein A Agarose/Salmon Sperm DNA beads (Upstate). 30 µl of supernatant were collected as input (I) sample. Chromatins were incubated with 3-5 µg of Ab (IP) or without Ab (B) overnight at 4°C. Immunoprecipitates were washed twice with buffer A (SDS 0,1 %, TRITON X-100 1%, EDTA 2 mM, Tris-HCl pH 8 20 mM, NaCl 150 mM), twice with buffer B (SDS 0,1 %, TRITON X-100 1%, EDTA 2 mM, Tris-HCl pH 8 20 mM, NaCl 500 mM), twice with buffer C (LiCl 0,25M,NP40 1%, deoxycholate 1 %, EDTA 1 mM, Tris-HCl pH 8 10 mM) and once with TE 1X. Immunocomplexes were eluted with 500 µl of elution buffer (SDS 1%, NaHCO3 0,1 M) at RT in rotation for 15'. The latter step was repeated twice. After crosslink reversion, proteins were extracted and DNA ethanol precipitated. The pellets were resuspended in 30 µl of H2O and amplified by PCR in the presence of [alfa-32P]dATP (1 microCi/12.5 microL). 1 µl of immunoprecipitated DNA (IP) and negative control without Ab (B-beads only) in 12,5 µl of total PCR reaction. Total input sample was diluted 1:10 before PCR. The promoter region of the following genes was analyzed: P-miR-206, P-miR-1, P-miR-29, P-HPRT1. Oligonucleotides for 5,8s rDNA were used as internal control by co-amplification with each of the specific primers. We analyzed the PCR products on a 8% acrylamide gel and visualized them using a Typhoon Imager (GE Healthcare).

Quantifications were performed with an ImageQuant software (Molecular Dynamics) and values normalized for internal control co-amplification and subtraction of background retained on the agarose beads (B). Each value in the histogram is the average of the signals on 3 different preparations of immunoprecipitated DNA.

### Results

6-week old *mdx* animals were tail vein injected with AAV recombinant viruses (AAV-U1#23) carrying a U1-chimeric antisense construct (Fig.1A) previously reported to induce the skipping of the mutated exon 23 and to restore dystrophin synthesis (Denti et al., 2006 and 2008). After 1 month, *mdx* and treated-*mdx* seeblings were sacrificed in parallel with wild type (WT) isogenic/aged matched animals. Different muscular districts were dissected and subdivided for RNA, protein and chromatin analysis. Body wide rescue (Denti et al., 2006) of dystrophin was obtained (Fig.1B,C). We took advantage of the intrinsic variability of *in vivo* transduction, following systemic delivery (Denti et al., 2006), in order to classify the animals on the basis of dystrophin rescue levels. Three different groups, each one including at least three different individuals, were obtained (G1, G2 and G3); compared to wild type mice, they display ≤1%, 1-5% and 5-10% of dystrophin rescue, respectively (Fig.1B). Even if these levels seem very low to confer any beneficial effect, it was demonstrated that amounts of dystrophin as low as a few percent are able to confer long life benefit to *mdx* muscles (Denti et al., 2008; Ghahramani Seno et al., 2008). Dystrophin rescue paralleled both the recovery of nNOS localization at the periphery of the fibers (Fig.1D) and morphological amelioration of the transduced muscle (Fig 1D). The effect was very significant in G3 animals but still detectable in G2 (not shown).

Real-time based low density arrays were performed on RNA from the gastrocnemius of wild type, *mdx,* and AAV-U1#23-treated *mdx* animals. miRNA expression levels, normalized for 5 endogenous controls (Fig. 8), revealed clear differences between WT and *mdx.* In the diagram of Fig. 2A, the miRNAs displaying the most significant variations (fold-change > 1,5) between WT and *mdx* are reported. Aware of the cellular heterogeneity of the dystrophic muscle, a parallel profile analysis on human DMD myoblasts (Fig. 9) was performed. Relevant differences between the animal and the *in vitro* cultured differentiated myoblasts were due to miRNAs belonging to cell types other than muscle ones (box B3 and B1) and, in particular as expected for a damaged dystrophic fiber, to miRNAs related to the inflammatory process (box B1, Fazi et al., 2005; Baltimore et al., 2008). Among the others, the B4 group contains miRNAs in common between cultured myoblasts and dissected muscles, while B2 include more ubiquitous miRNAs. The underlined miRNAs identify those species that in AAV-U1#23-treated *mdx* are recovered to levels similar to WT. Interestingly, this effect was proportional to the levels of dystrophin rescue, with G1 showing a very limited effect and G3 providing levels very close to the wild type ones (Fig. 8). Individual qRT-PCR were performed on B4 miRNAs and on a selection of control miRNAs. miR-1, miR-133a, miR-186, miR-29c and miR-30c, showing reduced levels in *mdx,* recover towards wild type levels in the three groups of treated animals proportionally to the level of dystrohin rescue (Fig. 2B). At difference with miR-1 and miR-133 myomiRs, miR-206 levels increase in *mdx* and in treated mice, while miR-31, displaying the strongest variation between WT and *mdx* did not show any variation in exon-skipped animals (not shown).

The same up-regulation of the above miRNAs was also observed in human DMD myoblasts treated with the exon skipping approach (Fig.2D and E).

Interestingly, the inflammatory-specific miR-223 (Fazi et al., 2005), very abundant in *mdx,* is proportionally reduced in the three different groups of animals, indicating the amelioration of the inflammatory state of the muscle due to dystrophin rescue (see fig.1c and Denti et al., 2008). The ubiquitous miR-23a and 27a, unchanged in WT and *mdx*, behaved similarly also in treated animals. Control injections of *mdx* mice with a AAV vector coding for the GFP protein indicated that the virus *per se* did not produce any change in miRNA levels (Fig. 2C). The relative expression of the selected miRNAs in WT, *mdx* and G3 animals was the same also in other muscle districts, demonstrating that the miRNA expression profile correlated with dystrophin rescue in a body-wide manner (Fig. 1C).

These data indicate that a specific subset of miRNAs undergoes altered expression in Duchenne Muscular Dystrophy as a direct consequence of dystrophin absence, while a different subset varies as a consequence of fiber damage.

Extracellular matrix proteins are known to increase in *mdx* animals where they contribute to the extensive fibrotic degeneration (Haslett et al., 2002). Crucial factors involved in this process are collagens and elastin whose mRNAs have been recently shown to be targets of the miR-29 family (van Rooij et al., 2008). qRT-PCR reveals that the mRNAs for these factors are up-regulated in *mdx,* where miR-29 is low, while significantly reduced in treated animals at levels similar to WT, where miR-29 is high (Fig. 3A). Therefore, the typical inverse correlation between miRNAs and corresponding targets is observed.

In order to test whether miR-29 alone is indeed responsible for this effect on collagen and elastin mRNAs, we electroporated in the gastrocnemius of *mdx* animals a plasmid DNA containing an expression cassette driving the transcription of miR-29a/b. Northern blot indicated that a 2-fold overexpression of miR-29 was specifically obtained in treated muscles which paralleled a decrease of collagen and elastin mRNAs (Fig. 3).

Trichromic staining analysis indicated a decrease in collagen deposition when muscles were electroporated with miR-29 in comparison to mock-treated controls (Fig. 3E). This suggest a direct role of miR-29 in the morphological benefit obtained in dystrophin-rescued animals. Therefore, both miR-29 or miR-30 can be utilized as therapeutic tool in order to ameliorate the fibrotic deposition consequent to muscle fiber degeneration. They can be administered either as synthetic molecules or as gene constructs with muscle specific promoters in the backbone of viral constructs. The delivery route can be either by local or systemic administration.

Chromatin immunoprecipitation (ChIP) analysis with antibodies against RNA polymerase II and H3K9 acetylation on the miR-1/133, miR-206 and miR-29 promoters (Fig. 4A) (Rao et al., 2006; Liu et al., 2007; Shkumatava et al., 2009; Wang et al., 2008, Han et al., 2004 and D.C. and M.C. personal data) correlated very well with the differential expression levels of the corresponding miRNAs in WT, *mdx* and G3 animals (Fig. 4B). Notably, in conditions where these miRNAs are repressed (*mdx* animals) a specific interaction was detected with HDAC2, while no reactivity was observed with either HDAC1 or HDAC4 (Fig. 4B). On the contrary, miR-206 and HPRT1 promoters, directing active transcription, do not show reactivity with any HDAC.

Interestingly, in growing C₂7 myoblasts, where miR-206 is poorly expressed (Fig. 5A,), a specific interaction was found between its promoter and HDAC1; upon differentiation, when transcription is activated, clearance of HDAC1 from the promoter was observed (Fig. 5B). In growth conditions, the miR-1/133 promoter was found again to be repressed by HDAC2 (Fig. 5A). The increased expression of miR-206 in *mdx* animals paralleled that of the satellite-specifying factor Pax 7 (Relaix, et al., 2006) (Fig.5C). These data, together with previous findings, reporting markedly increased expression of miR-206 in newly formed muscle fibers/myotubes (Yuasa et al., 2008) and a postulated role in regulating genes involved in satellite cells specification (McCarty, 2008), suggest a role of miR-206 in active regeneration and efficient maturation of skeletal muscle fibers. Notably, Pax7 and miR-206 levels are slightly higher in AAV-U1#23 treated animals (Fig.5C), indicating that dystrophin rescue parallels intensive muscle regeneration, in agreement with the morphological (Fig.1D) and functional benefit observed in treated animals (Denti et al., 2006). These results allow to conclude that miR-206 follows an epigenetic control independent from the one responsible for the expression of the terminally differentiating miR-1 and miR-133.

Due to the described correlation between nitrosylation and HDAC2 DNA binding (Nott et al., 2008), we tested whether the treatment of *mdx* animals with the NO donor, nitroglycerin (NTG), would provide the same epigenetic changes observed with the exon skipping treatment. Fig.6 shows that this treatment: i) up-regulates miR-1 and miR-29; ii) releases HDAC2 from the promoters of these miRNAs and iii) has no effect on miR-206 levels. Finally, it was tested that, as a consequence of muscle damage, specific muscle miRNAs are released into the blood. qRT-PCR on blood and serum of wild type versus dystrophic animals was performed. Fig.7 indicates that high levels of the muscle-specific miR-1 and miR-206 (15 and 30-fold increase respectively) are found in the serum of mdx animals. These results indicate that muscle damage in Duchenne individuals (as well as in other muscle degenerative disorders) can be easily and quantitatively assessed by measuring the serum levels of muscle specific miRNAs.

Altogether, these experiments clarify a new link between dystrophin and important genes encoding for miRNAs acting as regulators of muscle tissue differentiation and morphology. Moreover, the altered pattern of NO-dependent miRNAs in DMD can be extended to other muscle disorders in which the DAPC complex is absent or altered (such as in Girdle Limb dystrophy). These data also support the hypothesis that the morpho-functional benefit observed in exon-skipping *mdx* treated animals (Denti et al., 2008) may be due not only to the structural amelioration of the muscle membrane but also to an intracellular cascade of events controlling the expression of genes relevant for proper muscle homeostasis.

### References

1. Matsumura, K., et al. Expression of dystrophin-associated proteins in dystrophin-positive muscle fibers (revertants) in Duchenne muscular dystrophy. Neuromuscul. Disord. 4:115-120 (1994).
2. Ervasti, J.M., Sonnemann, K.J. Biology of the striated muscle dystrophin-glycoprotein complex. Int Rev Cytol. 265:191-225 (2008).
3. Denti, M.A., et al. Body-wide gene therapy of Duchenne Muscular Dystrophy in the mdx mouse model. Proc. Natl. Acad. Sci. USA 103:3758-3763 (2006).
4. Naguibneva I. et al. (2006) The microRNA miR-181 targets the homeobox protein Hox-A11 during mammalian myoblast differentiation. Nat Cell Biol.8 :278-84.
5. Chen J.F. et al. (2006) The role of microRNA-1 and microRNA-133 in skeletal muscle proliferation and differentiation. Nat Genet. 38:228-33.
6. Van Rooij, E., et al. Dysregulation of microRNAs after myocardial infarction reveals a role of miR-29 in cardiac fibrosis. Proc. Natl. Acad. Sci. USA 105:13027-13032 (2008).
7. Brenman, J.E., et al. Nitric oxide synthase complexed with dystrophin and absent from skeletal muscle sarcolemma in Duchenne muscular dystrophy. Cell 82:743-752 (1995).
8. Wehling, M., Spencer, M.J., Tidball, J.G. A nitric oxide synthase transgene ameliorates muscular dystrophy in mdx mice. J. Cell Biol. 155:123-131 (2001).
9. Riccio, A., et al. A nitric oxide signaling pathway controls CREB-mediated gene expression in neurons. Mol. Cell 21:283-294 (2006).
10. Nott, A., et al. S-nitrosylation of histone deacetylase 2 induces chromatin remodelling in neurons. Nature 455:411- 415 (2008).
11. Minetti, G.C., et al. Functional and morphological recovery of dystrophic muscles in mice treated with deacetylase inhibitors. Nat. Med. 12:1147-1150 (2006).
12. Brunelli, S., et al. Nitric oxide release combined with nonsteroidal antiinflammatory activity prevents muscular dystrophy pathology and enhances stem cell therapy. Proc. Natl. Acad. Sci. USA 104:264 -269 (2007).
13. Illi, B., et al. Nitric oxide modulates chromatin folding in human endothelial cells via protein phosphatase 2A activation and class II histone deacetylases nuclear shuttling. Circ Res. 102:51(2008).
14. Colussi, C., et al. Nitric oxide deficiency determines global chromatin changes in Duchenne muscular dystrophy. FASEB J. in press (2009).
15. Denti M. A., et al. Life-long benefit of AAV/antisense-mediated exon skipping in dystrophic mice. Hum. Gene Ther. 19:601-608 (2008).
16. Ghahramani Seno, M.M., et al. RNAi-mediated knockdown of dystrophin expression in adult mice does not lead to overt muscular dystrophy pathology. Hum. Mol. Genet. 17:2622-2632 (2008).
17. Eisenberg, I., et al. Distinctive patterns of microRNA expression in primary muscular disorders. Proc. Natl. Acad. Sci. USA 104:17016-17021 (2007).
18. Fazi, F., et al. A mini-circuitry comprising microRNA-223 and transcription factors NFI-A and C/EBPα regulates human granulopoiesis. Cell 123:819-831 (2005).
19. Baltimore, D., et al. MicroRNAs: new regulators of immune cell development and function. Nat. Immunol. 9:839-45 (2008).
20. Haslett, J.N., et al. Gene expression comparison of biopsies from Duchenne muscular dystrophy (DMD) and normal skeletal muscle. Proc. Natl. Acad. Sci. USA 99:15000-15005 (2002).
21. Liu, N., et al. An intragenic MEF2-dependent enhancer directs muscle-specific expression ofmicroRNAs 1 and 133. Proc. Natl. Acad. Sci USA 104:20844-20849 (2007).
22. Rao, P.K., et al. Myogenic factors that regulate expression of muscle-specific microRNAs. Proc. Natl. Acad. Sci. USA 103:8721-8726 (2006).
23. Relaix, F., et al. Pax3 and Pax7 have distinct and overlapping functions in adult muscle progenitor cells. J. Cell Biol. 172:91-102 (2006).
24. Shkumatava, A., Stark, A., Sive, H., Bartel, D.P. Coherent but overlapping expression of microRNAs and their targets during vertebrate development. Genes Dev. 23:466-81 (2009).
25. Wang, H., et al. NF-kappaB-YY1-miR-29 regulatory circuitry in skeletal myogenesis and rhabdomyosarcoma. Cancer Cell 14:369-381 (2008).
26. Lee, Y., et al. MicroRNA genes are transcribed by RNA polymerase II. EMBO J. 23: 4051-4060 (2004)
27. Yuasa, K., et al. MicroRNA-206 is highly expressed in newly formed muscle fibers: implications regarding potential for muscle regeneration and maturation in muscular dystrophy. Cell Struct. Funct. 33:163-169 (2008).
28. McCarthy, J.J., MicroRNA-206: the skeletal muscle-specific miomiR. Biochim. Biophys. Acta. 1779:682-691(2008).
29. Donà, M., et al. Functional in vivo gene transfer into the myofibers of adult skeletal muscle. Biochem. Biophys. Res. Commun. 312:1132-1138(2003).

## Claims

1. A method for the diagnosis of a muscle disorder and damage leading to muscle fiber degeneration, inflammation and fibrosis or for monitoring the progress of therapeutic treatments on subjects affected by said disorder consisting in detecting in a biological sample at least two of dystromiR molecules, each of the two dystromiR molecules belonging to one of the following groups: a) muscle regeneration (miR-206, miR-34), b) muscle differentiation (miR-1, miR-133), c) inflammatory (miR-223, miR-21) and d) tissue integrity (miR-29, miR-30).

2. The method according to claim 1 wherein said muscle disorder is the Duchenne Muscular Dystrophy.

3. The method according to claim 2 wherein the each of the two dystromiR molecules belongs to one of the following groups: a) muscle regeneration (miR-206, miR-34), b) muscle differentiation (miR-1, miR-133), c) inflammatory (miR-223, miR-21) and d) tissue integrity (miR-29, miR-30); Duchenne specific (miR-31).

4. The method according to claim 3 wherein the at least two of dystromiR molecules are selected in the following group: miR-31, miR-206, miR-1, miR-223 and miR-29.

5. The method according to any of previous claims wherein the biological sample is a muscle bioptic sample.

6. The method according to claims 1 to 4 wherein the biological sample is a serum or a blood sample.

7. The method according to any of previous claims wherein the detecting at least two of dystromiR molecules is performed by reverse amplification of said dystromiR molecules and real time detection of amplified products.

8. A kit to perform the method according to claim 4.

9. A dystromiR molecule belonging to the group of miR-29 and/or miR-30 for use as therapeutic medicament of Duchenne Muscular Dystrophy or of pathologies associated to mutations of DAPC components, such as Girdle Limb dystrophy, or as coadiuvant of other therapeutic strategies, such as exon skipping.

10. A pharmaceutical composition for local and/or systemic administration comprising the dystromiR molecule or a vector encoding the dystromiR molecule according to claim 9.

11. An agent able to functionally inactivate the dystromiR-31 molecule

12. The agent according to claim 11 as it can implement other as coadiuvant of other therapeutic strategies, such as exon skipping.
